# EUROPÄISCHE PATENTSCHRIFT

(11) **EP 1 314 714 B1**
(45) Veröffentlichungstag und Bekanntmachung des Hinweises auf die Patenterteilung: **11.07.2012**
(21) Anmeldenummer: 03004347.5
(22) Anmeldetag: 18.12.1998
(51) Int. Cl.: C07C 67/303, C07C 69/75, C07C 51/36, C07C 61/09, C08K 5/12

(54) **Verfahren zur Hydrierung von Benzolpolycarbonsäuren oder Derivaten davon unter Verwendung eines Makroporen aufweisenden Katalysators**
Method for hydrogenating benzene polycarboxylic acids or derivatives thereof by using a catalyst containing macropores
Procédé d'hydrogénation d'acides polycarboxyliques de benzène ou de leurs dérivés a l'aide d'un catalyseur a macropores

(30) Priorität: 19.12.1997 DE 19756913; 16.07.1998 DE 19832088
(43) Veröffentlichungstag der Anmeldung: 28.05.2003
(62) Teilanmeldung aus: 98966901.5
(73) Patentinhaber: BASF SE, 67056 Ludwigshafen (DE)
(72) Erfinder: Maas-Brunner, Melanie, Dr., 68165 Mannheim (DE); Böttcher, Arnd, Dr., 6727 Frankenthal (DE); Breitscheidel, Boris, Dr., 67117 Limburgerhof (DE); Halbritter, Klaus, Dr., 69124 Heidelberg (DE); Henkelmann, Jochen, Dr., 68165 Mannheim (DE); Thil, Lucien, Dr., 67117 Limburgerhof (DE); Pinkos, Rolf, Dr., 67098 Bad Dürkheim (DE)

(56) Entgegenhaltungen:
- EP-A- 0 005 738
- EP-A- 0 603 825
- DE-A- 2 823 165
- US-A- 5 286 898

## Beschreibung

Die vorliegende Erfindung betrifft ein Verfahren zur Hydrierung von Mono- oder Dialkylestern der Phthalsäure durch Inkontaktbringen eines oder mehrerer Mono-oder Dialkylester der Phthalsäure mit einem Wasserstoff enthaltenden Gas in Gegenwart eines Makroporen aufweisenden Katalysators.

In der US 5,286,898 und der US 5,319,129 wird Dimethylterephthalat an geträgerten Pd-Katalysatoren, die mit Ni, Pt und/oder Ru versetzt sind, bei Temperaturen ≥ 140 °C und einem Druck zwischen 50 und 170 bar zum entsprechenden Hexahydrodimethylterephthalat hydriert. In der DE-A 28 23 165 werden aromatische Carbonsäureester an geträgerten Ni-, Ru-, Rh-, und/ oder Pd-Katalysatoren zu den entsprechenden cycloaliphatischen Carbonsäure-estem bei 70 bis 250 °C und 30 bis 200 bar hydriert. In der US 3,027,398 wird die Hydrierung von Dimethylterephthalat an geträgerten Ru-Katalysatoren bei 110 bis 140 °C und 35 bis 105 bar beschrieben.

Die EP-A 0 603 825 betrifft ein Verfahren zur Herstellung von 1,4-Cyclohexandicarbonsäure durch Hydrierung von Terephthalsäure unter Verwendung eines geträgerten Palladium-Katalysators, wobei als Träger Aluminiumoxid, Siliciumdioxid oder Aktivkohle verwendet wird. Das dort beschriebene Verfahren ist insbesondere dadurch charakterisiert, daß die in einer ersten Stufe erhaltene 1,4-Cyclohexandicarbonsäure enthaltende Lösung mit Dampf in Kontakt gebracht wird und dadurch in dieser Lösung enthaltene Verunreinigungen extrahiert werden. Dieses Verfahren ist jedoch nur auf Säuren anwendbar, da bei der Anwendung auf Derivate, wie z.B. Ester, Anhydride, usw. die Gefahr von Hydrolyse besteht. Die Verwendung eines Makroporen aufweisenden Trägers wird in dieser Anmeldung mit keinem Wort erwähnt.

DE 28 23 165 offenbart ein Verfahren zur Herstellung von cycloaliphatischen Carbonsäureestern aus den entsprechenden aromatischen Carbonsäureestern mit Hilfe eines Nickel- und/oder Ruthenium- und/oder Rhodium- und/oder Palladiumkatalysators.

Bislang wurden als Weichmacher in Kunststoffen, wie z.B. PVC sehr häufig Phthalsäureestern, wie z.B. Dibutyl-, Dioctyl- oder Diisononylester der Phthalsäure verwendet, wie dies z.B. aus der FR-A 23 97 131 hervorgeht. Diesen wird jedoch seit kurzer Zeit nachgesagt, daß sie gesundheitlich nicht unbedenklich sind, sodaß ihre Verwendung in Kunststoffen zur Verwendung von z.B. Kinderspielzeug immer stärker in der Kritik steht und in einigen Ländern bereits verboten ist.

Die Verwendung von einigen Cyclohexan-1,2-dicarbonsäureestern als Weichmacher ist ebenfalls aus dem Stand der Technik bekannt. So sind die Verwendung von Cyclohexandicarbonsäuredimethyl oder -diethylester (DE-A 28 23 165) und Cyclohexan-1,2-dicarbonsäuredi(2-ethylhexyl)ester (DE-A 12 63 296), als Weichmacher in Kunstoffen beschrieben.

Der vorliegenden Erfindung lag die primäre Aufgabe zugrunde, ein Verfahren zur Hydrierung von Benzolpolycarbonsäure(derivate)n, insbesondere Benzoldicarbonsäureestern unter Verwendung spezifischer Katalysatoren zur Verfügung zu stellen, mit deren Hilfe die entsprechenden kernhydrierten Derivate, insbesondere Cyclohexandicarbonsäureester mit sehr hoher Selektivität und Raum-Zeit-Ausbeute ohne signifikante Nebenreaktionen erhalten werden können.

Demgemäß betrifft die vorliegende Erfindung ein Verfahren zur Hydrierung eines Mono- und Dialkylesters der Phthalsäure, wobei die Alkylgruppen linear oder verzweigt sein können und jeweils 3 bis 18 Kohlenstoffatome aufweisen, oder eines Gemischs aus zwei oder mehr davon durch Inkontaktbringen des Mono- und Dialkylesters der Phthalsäure oder des Gemischs aus zwei oder mehr davon mit einem Wasserstoff-enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfasst, dadurch gekennzeichnet, dass der Träger Makroporen mit einem Porendurchmesser oberhalb von 50 nm gemäß der Definition in Pure Applied Chemistry, 46, S. 79 (1976) aufweist, wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfasst, wobei der Träger Makroporen aufweisendes Aluminiumoxid ist und einen mittleren Porendurchmesser von mindestens 0,1 µm, und eine BET-Oberfläche von höchstens 15 m²/g aufweist, und mit der Maßgabe, dass solche Katalysatoren ausgeschlossen sind, die als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfassen, wobei 5 bis 50% des Porenvolumens des Trägers von Makroporen mit einem Porendurchmesser im Bereich von 50 nm bis 10.000 nm und 50 bis 95 % des Porenvolumens des Trägers von Mesoporen mit einem Porendurchmesser im Bereich von 2 bis 50 nm gebildet werden, wobei sich die Summe der Anteile Porenvolumina zu 100% addiert.

Erfindungsgemäß ausgeschlossen sind Katalysatoren, die als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfassen, wobei 5 bis 50 % des Porenvolumens des Trägers von Makroporen mit einem Porendurchmesser im Bereich von 50 nm bis 10.000 nm und 50 bis 95 % des Porenvolumens des Trägers von Mesoporen mit einem Porendurchmesser im Bereich von 2 bis 50 nm gebildet werden, wobei sich die Summe der Anteile der Porenvolumina zu 100% addiert.

Die vorliegende Erfindung betrifft ein Verfahren, wie oben definiert, wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei der Träger Makroporen aufweisendes Aluminiumoxid ist und einen mittleren Porendurchmesser von mindestens 0,1 µm, und eine BET-Oberfläche von höchstens 15 m²/g aufweist. Als Träger können prinzipiell alle Träger eingesetzt werden, die Makroporen aufweisen, d.h. Träger, die ausschließlich Makroporen aufweisen sowie solche, die neben Makroporen auch Meso- und/oder Mikroporen enthalten.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe *"Makroporen"* und *"Mesoporen"* werden im Rahmen der vorliegenden Erfindung so verwendet, wie sie in Pure Appl. Chem., 46, S. 79 (1976*)* definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei dem im folgenden beschriebene, vorzugsweise eingesetzten Katalysator vorzugsweise verwendeten Gehalte nochmals bei der Diskussion dieses Katalysators einzeln angegeben sind.

Im folgenden soll nunmehr der vorzugsweise verwendete Katalysator detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

Die erfindungsgemäß verwendeten Katalysatoren können technisch hergestellt werden durch Auftragen eines Aktivmetalls der VIII. Nebengruppe des Periodensystems und gegebenenfalls mindestens eines Metalls der I. oder VII. Nebengruppe des Periodensystems auf einen geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen von 100 bis 150 °C, und wahlweise bei Temperaturen von 200 bis 600 °C calciniert.

Darauffolgend werden die beschichteten Träger aktiviert durch Behandlung der beschichteten Träger in einem Gasstrom, der freien Wasserstoff enthält, bei Temperaturen von 30 bis 600 °C, vorzugsweise von 150 bis 450 °C. Der Gasstrom besteht vorzugsweise aus 50 bis 100 Vol-% H₂ und 0 bis 50 Vol-% N₂.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, daß 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 m²/g, besonders bevorzugt 0,05 bis 5 m²/g, insbesondere 0,05 bis 3 m² pro g des Katalysators.

Die zur Herstellung der erfindungsgemäß verwendeten Katalysatoren verwendbaren Trägermaterialien sind solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial wird Makroporen aufweisendes Aluminiumoxid verwendet.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

### DIE VERFAHRENSFÜHRUNG

Im Rahmen des erfindungsgemäßen Verfahrens wird die Hydrierung im allgemeinen bei einer Temperatur von ungefähr 50 bis 250 °C, vorzugsweise ungefähr 70 bis 220 °C durchgeführt. Die dabei verwendeten Drücke liegen in der Regel bei oberhalb von 10 bar, vorzugsweise ungefähr 20 bis ungefähr 300 bar.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge des Mono- und Dialkylesters der Phthalsäure bzw. des Gemischs aus zwei oder mehr davon vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die erfindungsgemäße Hydrierung kann in Ab- oder Anwesenheit eines Lösungs-oder Verdünnungsmittels durchgeführt werden, d.h. es ist nicht erforderlich, die Hydrierung in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit der (dem) zu hydrierenden Benzoldicarbonsäure(ester) eine homogene Lösung zu bilden. Beispielsweise können die Lösungs- oder Verdünnungsmittel auch Wasser enthalten.

Beispiele geeigneter Lösungs- oder Verdünnungsmittel schließen die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung der (des) zur Hydrierung vorgesehenen Benzoldicarbonsäure(esters) führen.

Besonders bevorzugt wird im Rahmen des erfindungsgemäßen Verfahrens das bei der Hydrierung gebildete Produkt, also das entsprechende Cyclohexanderivat als Lösungsmittel eingesetzt, gegebenenfalls neben anderen Lösungs- oder Verdünnungsmitteln. In jedem Fall kann ein Teil des im Verfahren gebildeten Produkts der noch zu hydrierenden Benzolpolycarbonsäure oder des Derivats davon beigemischt werden. Bezogen auf das Gewicht der zur Hydrierung vorgesehenen Verbindung wird vorzugsweise die 1- bis 30fache, besonders bevorzugt die 5- bis 20fache, insbesondere die 5- bis 10fache Menge des Umsetzungsproduktes als Lösungs- oder Verdünnungsmittel zugemischt.

Die eingesetzten Ester sind Alkylester, wobei die Alkylgruppen 3 bis 18 Kohlenstoffatome umfassen und verzweigt oder linear sein können.

Im einzelnen sind zu nennen:
Phthalsäurealkylester, wie z.B. Phthalsäuredi-n-propylester, Phthalsäuredi-n-butylester, Phthalsäuredi-tert.-butylester, Phthalsäuredüsobutylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäurediisodecylester, Phthalsäuredi-n-undecylester, Phthalsäurediisododecylester, Phthalsäuredi-n-octadecylester, Phthalsäuredüsooctadecylester, Phthalsäuredi-n-eicosylester, Phthalsäuremonocyclohexylester, Phthalsäuredicyclohexylester.

Selbstverständlich können auch Gemische aus zwei oder mehr dieser Verbindungen eingesetzt werden.

Bei den erfindungsgemäß erhaltenen Produkten handelt es sich dabei stets um die entsprechenden Cyclohexanpolycarbonsäuren.

Verglichen mit den bislang hauptsächlich als Weichmacher verwendeten Phthalaten besitzen die erfindungsgemäß hergestellten Cyclohexanpolycarbonsäure(derivate) eine niedrigere Dichte und Viskosität und führen u.a. zu einer Verbesserung der Kälteflexibilität des Kunststoffs gegenüber der Verwendung der entsprechenden Phthalate als Weichmacher, wobei Eigenschaften wie Shore A-Härte und mechanische Eigenschaften der resultierenden Kunststoffe identisch zu denen sind, die bei Verwendung von Phthalaten resultieren. Ferner besitzen die erfindungsgemäß hergestellten Cyclohexanpolycarbonsäure(derivate) ein besseres Verarbeitungsverhalten im Dry-Blend und als Folge eine erhöhte Produktionsgeschwindigkeit sowie in Plastisol-Verarbeitungen Vorteile durch eine deutlich niedrigere Viskosität gegenüber den entsprechenden Phthalaten.

## Patentansprüche

1. Verfahren zur Hydrierung eines Mono- und Dialkylesters der Phthalsäure, wobei die Alkylgruppen linear oder verzweigt sein können und jeweils 3 bis 18 Kohlenstoffatome aufweisen, oder eines Gemischs aus zwei oder mehr davon durch Inkontaktbringen des Mono- und Dialkylesters der Phthalsäure oder des Gemischs aus zwei oder mehr davon mit einem Wasserstoff - enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, **dadurch gekennzeichnet, dass** der Träger Makroporen mit einem Porendurchmesser oberhalb von 50 nm gemäß der Definition in Pure Applied Chemistry, 46, S. 79 (1976) aufweist,
wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei der Träger Makroporen aufweisendes Aluminiumoxid ist und einen mittleren Porendurchmesser von mindestens 0,1 µm, und eine BET-Oberfläche von höchstens 15 m²/g aufweist,
und mit der Maßgabe,
dass solche Katalysatoren ausgeschlossen sind, die als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfassen, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Anteile Porenvolumina zu 100% addiert.

2. Verfahren nach Anspruch 1, **dadurch gekennzeichnet, dass** die Hydrierung in Gegenwart eines Lösungs- oder Verdünnungsmittels durchgeführt wird.

3. Verfahren nach einem der vorstehenden Ansprüche, **dadurch gekennzeichnet, dass** die Hydrierung kontinuierlich durchgeführt wird.

## Claims

1. A process for hydrogenating a mono- or dialkyl phthalate, where the alkyl groups can be linear or branched and each have from 3 to 18 carbon atoms, or a mixture of two or more thereof by bringing the mono- or dialkyl phthalate or the mixture of two or more thereof into contact with a hydrogen-comprising gas in the presence of a catalyst which comprises as active metal at least one metal of transition group VIII of the Periodic Table either alone or together with at least one metal of transition group I or VII of the Periodic Table applied to a support, wherein the support contains macropores having a pore diameter above 50 nm as defined in Pure Applied Chemistry, 46, p. 79 (1976),
where the catalyst comprises as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where the support is aluminum oxide containing macropores and has a mean pore diameter of at least 0.1 µm and a BET surface area of at most 15 m²/g,
and with the proviso
that those catalysts are excluded which comprise as active metal at least one metal of transition group VIII of the Periodic Table either alone or together with at least one metal of transition group I or VII of the Periodic Table in an amount of from 0.01 to 30% by weight, based on the total weight of the catalyst, applied to a support, where from 5 to 50% of the pore volume of the support is formed by macropores having a pore diameter in the range from 50 nm to 10 000 nm and from 50 to 95% of the pore volume of the support is formed by mesopores having a pore diameter in the range from 2 to 50 nm, where the sum of the pore volumes adds up to 100%.

2. The process according to claim 1, wherein the hydrogenation is carried out in the presence of a solvent or diluent.

3. The process according to either of the preceding claims, wherein the hydrogenation is carried out continuously.

## Revendications

1. Procédé pour l'hydrogénation d'un ester mono-et dialkylique de l'acide phtalique, les groupes alkyle pouvant être linéaires ou ramifiés et comportant chacun de 3 à 18 atomes de carbone, ou d'un mélange de deux ou plus de deux de tels esters, par mise en contact de l'ester mono- et dialkylique de l'acide phtalique, ou du mélange de deux ou plus de deux de tels esters, avec un gaz contenant de l'hydrogène, en présence d'un catalyseur qui comprend comme métal actif au moins un métal du groupe VIIIB du système périodique, seul ou conjointement avec au moins un métal du groupe IB ou VIIB du système périodique, appliqué sur un support, **caractérisé en ce que** le support comporte des macropores ayant un diamètre de pore supérieur à 50 nm selon la définition dans *Pure Applied Chemistry,* 46, p. 79 (1976),
dans lequel le catalyseur comprend comme métal actif au moins un métal du groupe VIIIB du système périodique, seul ou conjointement avec au moins un métal du groupe IB ou VIIB du système périodique en une quantité de 0,01 à 30 % en poids, par rapport au poids total du catalyseur, appliqué sur un support, le support étant de l'oxyde d'aluminium qui comporte des macropores et présentant un diamètre moyen de pore d'au moins 0,1 µm, et une surface BLET d'au maximum 15 m²/g,
étant entendu
que sont exclus les catalyseurs qui comprennent comme métal actif au moins un métal du groupe VIIIB du système périodique, seul ou conjointement avec un métal du groupe IB ou VIIB du système périodique, en une quantité de 0,01 à 30 % en poids, par rapport au poids total du catalyseur, appliqué sur un support, 5 à 50 % du volume de pores du support étant constitués par des macropores ayant un diamètre de pore dans la plage de 50 nm à 10 000 nm et 50 à 95 % du volume de pores du support étant constitués par des mésopores ayant un diamètre de pore dans la plage de 2 à 50 nm, la somme des proportions de volumes de pores étant égale à 100 %.

2. Procédé selon la revendication 1, **caractérisé en ce que** l'hydrogénation est effectuée en présence d'un solvant ou diluant.

3. Procédé selon l'une quelconque des revendications précédentes, **caractérisé en ce que** l'hydrogénation est effectuée en continu.
